# EUROPÄISCHE PATENTSCHRIFT

(11) **EP 3 558 599 B1**
(45) Veröffentlichungstag und Bekanntmachung des Hinweises auf die Patenterteilung: **25.11.2020**
(21) Anmeldenummer: 17829125.8
(22) Anmeldetag: 15.12.2017
(51) Int. Cl.: B25J 9/16, A61B 34/30, A61B 34/20, A61B 90/00

(54) **VERFAHREN ZUM KALIBRIEREN EINES MANIPULATORS EINES DIAGNOSTISCHEN UND/ODER THERAPEUTISCHEN MANIPULATORSYSTEMS**
METHOD FOR CALIBRATING A MANIPULATOR OF A DIAGNOSTIC AND/OR THERAPEUTIC MANIPULATOR SYSTEM
PROCÉDÉ D'ÉTALONNAGE D'UN MANIPULATEUR D'UN SYSTÈME DE MANIPULATEUR DIAGNOSTIQUE ET/OU THÉRAPEUTIQUE

(30) Priorität: 20.12.2016 DE 102016225613
(43) Veröffentlichungstag der Anmeldung: 30.10.2019
(73) Patentinhaber: KUKA Deutschland GmbH, 86165 Augsburg (DE)
(72) Erfinder: KELLER, Henrik, 40219 Düsseldorf (DE); MEWES, Philip, 90419 Nürnberg (DE)
(74) Vertreter: Tillmann, Axel
(86) Internationale Anmeldenummer: PCT/EP2017/001428
(87) Internationale Veröffentlichungsnummer: WO 2018/114041

(56) Entgegenhaltungen:
- EP-A1- 1 932 488
- EP-A1- 3 295 339
- EP-A1- 3 329 877
- EP-A1- 3 491 367
- WO-A1-2016/009339
- WO-A1-2018/020009
- US-A- 5 408 409
- US-A1- 2009 326 365
- US-A1- 2016 360 947

## Beschreibung

### Gebiet der Erfindung

Die Erfindung betrifft ein Verfahren zum Kalibrieren eines Manipulators eines diagnostischen und/oder therapeutischen Manipulatorsystems.

### Hintergrund der Erfindung

Diagnostische und/oder therapeutische Manipulatorsysteme sind aus dem Stand der Technik bekannt und umfassen zumindest einen Manipulator, der zu diagnostischen und/oder therapeutischen Zwecken genutzt werden kann. Manipulatoren sind Geräte, welche die physikalische Interaktion mit der Umgebung ermöglichen. Typische Manipulatoren sind Industrie- oder Gelenkarmroboter, die drei oder mehr frei programmierbare Bewegungsachsen (Glieder) umfassen, welche automatisch geführt werden können. Diese Manipulatoren werden entweder ortsfest oder mobil in unterschiedlichen Anwendungen eingesetzt. Insbesondere sind sie dazu eingerichtet Endeffektoren oder Werkstücke zu führen. Endeffektoren können unterschiedliche Werkzeuge sein. Im Feld der diagnostischen und/oder therapeutischen Manipulatorsysteme können Endeffektoren beispielsweise chirurgische Instrumente, Skalpelle, Nadelhalter zum Anfertigen chirurgischer Nähte, Systeme zum Einführen von Biopsienadeln, Trokare und dergleichen umfassen. Weitere Beispiele für Endeffektoren sind Endoskope, Stapler, usw.

Manipulatoren und insbesondere Gelenkarmroboter werden in immer größerer Anzahl im medizinischen Umfeld eingesetzt. Sie kommen unter anderem in automatisierten Manipulatorsystemen, teilautomatisierten Manipulatorsystemen und teleoperativen Manipulatorsystemen zum Einsatz. In automatisierten bzw. teilautomatisierten Manipulatorsystemen werden (Teil-)aufgaben von dem Manipulator eigenständig ausgeführt. In teleoperativen Manipulatorsystemen wird der Manipulator mittels geeigneter Eingabevorrichtungen von einem Operateur manuell gesteuert. Der Manipulator setzt die Eingabebefehle der Eingabevorrichtung in entsprechende Manipulatorbewegungen um. Operateur und Manipulator können dabei räumlich getrennt sein.

Beispielsweise werden auf dem Feld der Gewebebiopsie teilautomatisierte Manipulatorsysteme eingesetzt, um eine Biopsienadel so zu positionieren und zu orientieren, dass beim Vorschieben der Biopsienadel, d.h. beim Stechen, das zu biopsierende Gewebe sicher getroffen wird. Das Vorschieben kann dabei händisch oder mittels teleoperativer Systeme erfolgen. Andere bekannte Anwendungen betreffen das Setzen von Implantaten, insbesondere das Setzen von chirurgischen Schrauben.

Die therapeutischen und/oder diagnostischen Anwendungen werden oftmals mittels medizinischer Bildgebungsvorrichtungen überwacht und/oder geplant. So muss beispielsweise beim Setzen chirurgischer Schrauben überwacht werden, dass die Schraube keine Nerven oder andere Gewebestrukturen des Patienten ungewollt verletzt. Bekannte Bildgebungsvorrichtungen liefern (zwei- oder dreidimensionale) Bilddaten von Organen und Strukturen des Patienten. Systematisieren lassen sich Bildgebungsvorrichtungen nach der Art ihrer Bilderzeugung mittels Röntgenstrahlung (z. B. Röntgengeräte, C-Bögen, Computertomographie), Radionukliden (z. B. Szintigraphie, Positronen-Emissions-Tomographie, Single-Photon-Emissionscomputertomographie), Ultraschall (z. B. Sonographie, Farbdoppler) und Kernspinresonanz (z. B. Magnetresonanztomographie). Ebenso kann die Bildgebungsvorrichtung optische Sensoren, wie Kameras, umfassen, die Bilder basierend auf sichtbarem Licht erzeugen.

Manipulator geführte Werkzeuge (Endeffektoren) haben gegenüber rein händisch geführten Werkzeugen den Vorteil, dass sie wiederholt positionsgenau eingesetzt werden können, ohne dabei von menschlichen Fehlerquellen wie Tremor, nachlassende Konzentration, Müdigkeit und dergleichen abhängig zu sein. Um eine hohe Genauigkeit des Manipulators und insbesondere des Endeffektors zu erzielen, werden Manipulatoren typischerweise kalibriert und/oder justiert.

Bei der Bestimmung der Genauigkeit eines Manipulators wird zwischen Wiederholgenaugkeit und Absolutgenauigkeit unterschieden. Die Absolutgenauigkeit gibt die Abweichung zwischen der Ist-Pose des Endeffektors und der erwarteten Soll-Pose des Endeffektors an, wobei die Posen typischerweise relativ zu einem externen Referenz-Koordinatensystems angegeben sind. Beispielsweise wird das Manipulator-Basiskoordinatensystem zugrunde gelegt. Die Absolutgenauigkeit eines Manipulators wird meist, durch Vermessung der mittleren oder maximalen Abweichung zwischen der Soll-Pose und der Ist-Pose bestimmt, die sich beim Anfahren der Soll-Pose aus unterschiedlichen Richtungen (multidirektional) ergeben. Eine Pose umfasst die Position und Orientierung des Endeffektors.. Die Wiederholgenauigkeit gibt an, wie genau ein Manipulator bei mehrfachem Anfahren einer Pose aus der gleichen Richtung positioniert, und ist als die Abweichung zwischen den erreichten Ist-Posen zu bewerten. Die Wiederholgenauigkeit kann ohne Kenntnis der genauen Lage des Basiskoordinatensystems gemessen werden, da keine Soll-Pose zum Vergleich herangezogen werden muss.

Beide Genauigkeiten werden durch unterschiedliche Faktoren negativ beeinflusst. Insbesondere Achsnulllagen sowie die Längen- und Winkelfehlern zwischen den einzelnen Gliedern des Manipulators wirken sich negativ auf die Genauigkeit aus. Weitere Fehlerquellen sind unter anderem variierende Lasten sowie Verschleiß und Erwärmung. Bei der Verwendung von Manipulatoren in diagnostischen und/oder therapeutischen Anwendungen muss der Manipulator eine hohe Genauigkeit (insbesondere eine hohen Absolutgenauigkeit) aufweisen, um die Gefährdung des Patienten so gering wie möglich zu halten oder um spezifische Anwendungen, wie beispielsweise die Mikrochirurgie, überhaupt erst zu ermöglichen.

Es ist bekannt, dass sowohl die Wiederholgenauigkeit als auch die Absolutgenauigkeit durch eine Kalibrierung des Roboters erhöht werden können. Die Faktoren, die die Genauigkeit negativ beeinflussen, werden dazu vermessen und in kinematischen Modellen oder Korrekturmatrizen erfasst, um beim Verfahren des Manipulators als Kalibrierungsparameter zum Einsatz zu kommen. Die erhöhte Genauigkeit, die mittels der Kalibrierung erreicht wird, nimmt mit der Zeit, beispielsweise durch Getriebeabnutzung, mechanische Ermüdung oder extreme Benutzungsszenarien (z.B. Not-Stopp bei hoher Geschwindigkeit) ab, wodurch eine erneute Kalibrierung erforderlich wird.

Zur Kalibrierung des Manipulators können verschiedene Messsysteme zum Einsatz kommen. Diese umfassen Systeme der Laserinterferometrie, Theodoliten, Messtaster oder Lasertriangulationsvorrichtungen. Diese Messsysteme zeichnen sich durch eine sehr hohe Messgenauigkeit aus, sind aber üblicherweise teuer und nicht ohne weiteres zu transportieren, wodurch der Einsatz im medizinischen Umfeld, wie beispielsweise in einem Operationssaal, erschwert oder sogar unmöglich wird.

Insbesondere ist eine Kalibrierung notwendig, wenn durch den Transport des Manipulators zum Einsatzort eine Kalibrierung, die z.B. bei der Produktion des Manipulators durchgeführt wurde, nicht mehr valide ist und/oder aufgrund der Montage des Manipulators am Einsatzort neue oder geänderte, z.B. geometrische Parameter, in die Kalibrierung einfließen müssen. Weiterhin kann der Austausch von Ersatzteilen oder das Auftreten extremer Benutzungsszenarien, wie Not-Stopps o.ä., eine erneute Kalibrierung erforderlich machen. Eine Kalibrierung am Einsatzort ist folglich oft unumgänglich und verursacht hohe Kosten, da Messsysteme an den Einsatzort des Manipulators verbracht und dort in Betrieb genommen werden müssen.

Die Druckschrift US 2016/360947 A1 offenbart ein medizinisches System mit einem Endoskop mit den Merkmalen des Oberbegriffs des Anspruchs 1. Die Druckschrift US 5 408 409 A offenbart ein bildgeführtes Robotersystem für Präzisionschirurgie. Die Druckschrift EP 1 932 488 A1 offenbart einen Rahmen für ein System zur Referenzerfassung.

Aufgabe der vorliegenden Erfindung ist es die zuvor genannten Nachteile zumindest teilweise auszuräumen und ein Verfahren zur Kalibrierung eines Manipulators eines diagnostischen und/oder therapeutischen Manipulatorsystems bereitzustellen, das genau ist und einfach am Einsatzort des Manipulators ausgeführt werden kann.

### Ausführliche Beschreibung der Erfindung

Die Aufgabe wird durch ein Verfahren zum Kalibrieren eines Manipulators nach Anspruch 1 oder durch ein computerlesbares Medium nach Anspruch 13 gelöst.

Insbesondere wird die Aufgabe durch ein Verfahren zum Kalibrieren eines Manipulators eines diagnostischen und/oder therapeutischen Manipulatorsystems gelöst, wobei das Manipulatorsystem zumindest eine medizinische Bildgebungsvorrichtung umfasst, und wobei das Verfahren zumindest die folgenden Schritte aufweist:
a) Anfahren zumindest einer Soll-Pose mittels des Manipulators (10);
b) Erfassen zumindest eines Bildes zumindest eines Teils des Manipulators und/oder zumindest eines Teils eines Endeffektors des Manipulators mittels der medizinischen Bildgebungsvorrichtung, wenn der Manipulator die Soll-Pose angefahren hat;
c) Bestimmen der Ist-Pose des Manipulators mittels des erfassten Bildes;
d) Bestimmen der Abweichung zwischen Soll-Pose und Ist-Pose des Manipulators;
e) Berechnen zumindest eines Kalibrierungsparameters basierend auf der bestimmten Abweichung, und
f) Kalibrieren des Manipulators.

Das Anfahren der zumindest einen Soll-Pose mittels des Manipulators kann von verschiedenen Richtungen erfolgen, um möglichst genaue Kalibrierungsparameter berechnen zu können.

Das Erfassen des Bildes mittels der medizinischen Bildgebungsvorrichtung erfolgt derart, dass zumindest ein Teil des Manipulators und/oder des Endeffektors auf dem

Bild erfasst sind. Dies bedeutet, dass die Soll-Pose entweder so gewählt werden muss, dass zumindest ein Teil des Manipulators und/oder ein Teil des Endeffektors in den Bilderfassungsbereich der Bildgebungsvorrichtung hineinragt, wenn der Manipulator die Soll-Pose annimmt, oder dass die Bildgebungsvorrichtung entsprechend positioniert und orientiert werden muss, um den Manipulator/den Endeffektor in der Soll-Pose erfassen zu können.

Die Soll-Pose ist dabei typischerweise über den Tool-Center-Point des Endeffektors definiert und beschreibt Position und Orientierung des Endeffektors im Raum. Kommen redundante Manipulatoren zum Einsatz, d. h. Manipulatoren die typischerweise mehr als sechs Freiheitsgrade aufweisen, so können zur eindeutigen Bestimmung der Soll-Pose weitere Parameter notwendig sein. Entsprechend können beim Erfassen des Bildes auch mehrere Teile des Manipulators oder der gesamte Manipulator erfasst werden.

Insbesondere ist unter Erfassen zumindest eines Bildes mittels der medizinischen Bildgebungsvorrichtung zu verstehen, dass die medizinische Bildgebungsvorrichtung derart zur Bilderzeugung genutzt wird, als ob die medizinische Bildgebungsvorrichtung Bilder eines Patienten zur Diagnose oder Therapie erstellen würde. Dies schließt jedoch nicht aus, dass die medizinische Bildgebungsvorrichtung zum Erfassen des Bildes derart betrieben wird, dass die Ist-Pose des Manipulators besonders gut, ein Patient hingegen schlechter erkannt werden kann. Beispielsweise kann ein MRT zur Erfassung des Bildes in einem ersten Modus betrieben werden, welcher den Manipulator, Endeffektor oder etwaige Marker besonderes deutlich erfasst. Zur Erfassung des Patienten, d.h. zur eigentlichen medizinischen Bildgebung, kann das MRT dann in einem zweiten Modus betrieben werden.

Weiterhin kommt es nicht darauf an, dass das erfasste Bild tatsächlich in Form eines visuellen Eindrucks (z.B. als Foto, oder dergleichen) an einen Benutzer/Bediener oder an eine das Verfahren ausführende Einrichtung übermittelt wird. Vielmehr umfasst das Erfassen des Bildes das Erfassen von jeglichen Bildrohdaten, die dazu geeignet sind in für die medizinische Bildgebungsvorrichtung typische Bilder gewandelt zu werden.

Soll beispielsweise als medizinische Bildgebungsvorrichtung ein Ultraschallsystem zum Einsatz kommen, welches Bilder anhand von Laufzeitmessdaten der Ultraschallwellen erstellt, so ist es für das Verfahren ausreichend, dass die entsprechenden Rohdaten, d.h. die Laufzeitmessdaten erfasst werden. Ebenso ist es beispielsweise in auf Magnetresonanz basierenden Bildgebungssystemen ausreichend, die Rohdaten des Magnetresonanztomographen zu erfassen, welche geeignet sind in entsprechende Bilddaten gewandelt zu werden. Beim Erfassen des zumindest einen Bildes mittels der Bildgebungsvorrichtung kommt es folglich nicht darauf an ein tatsächliches Bild im Sinne eines Fotos oder eines visuellen Eindruckes zu erzeugen, sondern es genügt die den bildgebungsvorichtungsspezifischen Bildern zugrundeliegenden Rohdaten zu erfassen und entsprechend dem Verfahren weiter zu bearbeiten. Die Bildgebungsvorrichtungen die im Verfahren zum Einsatz kommen können, sind nicht beschränkt und umfassen die eingangs erwähnten typischen Bildgebungsvorrichtungen, die im medizinischen Umfeld zum Einsatz kommen.

Basierend auf dem erfassten Bild wird die Ist-Pose des Manipulators bestimmt und eine Abweichung zwischen Soll-Pose und Ist-Pose bestimmt. Die Abweichung ist ein Maß für die absolute Genauigkeit. Soll die Wiederholgenauigkeit verbessert werden, so kann die Soll-Pose eine zuvor angefahrene Pose sein und muss nicht durch exakte Koordinaten im Raum bestimmt sein.

Basierend auf den bestimmten Abweichungen werden dann Kalibrierungsparameter berechnet, die wiederum genutzt werden, um den Manipulator zu kalibrieren. Typischerweise wird dies erzielt indem ein Model des Manipulators in einer Steuereinrichtung des Manipulators angepasst wird, sodass kommandierte Steuerbefehle zum exakten/genauen Anfahren der Soll-Pose führen.

Um die Ist-Pose zu bestimmen, muss die Position und Orientierung von der Bildgebungsvorrichtung relativ zum Manipulator bekannt sein. Dazu kann der Manipulator in einem bekannten Abstand bzw. in einer bekannten Orientierung zur Bildgebungsvorrichtung angeordnet sein. Dies kann beispielsweise erreicht werden, indem der Manipulator fest mit der Bildgebungsvorrichtung verbunden ist oder indem der Manipulator und die Bildgebungsvorrichtung an einer bekannten Position fest verankert werden.

Ebenso ist es möglich Marker vorzusehen, wie weiter unten beschrieben, die beispielsweise ortsfest angeordnet sind. Auch kann über bestimmte Stellungen des Endeffektors auf die Position und Orientierung der Manipulatorbasis geschlossen werden. Wird der Manipulator beispielsweise vollständig gestreckt (Kerzenstellung) und der Endeffektor des Manipulators bzw. das letzte Glied des Manipulators erfasst, so kann auf die Lage der Manipulatorbasis rückgeschlossen werden, wenn die Geometrie des Manipulators in Kerzenstellung bekannt ist. Dies erlaubt eine Steigerung der Genauigkeit, d. h. eine Kalibrierung, da typischerweise derartige Stellungen einen sehr kleinen Stellungsfehler, d. h. Abweichungen in der Pose aufweisen. Weiterhin kann die Position und Orientierung von der Bildgebungsvorrichtung relativ zum Manipulator durch sogenanntes Referenzieren oder Handführen des Manipulators bestimmt werden. Dazu wird beispielsweise der Endeffektor händisch zu einem Punkt bekannter Koordinaten (Referenzpunkt) geführt. Der Manipulator kann dazu tatsächlich manuell bewegt werden oder über eine geeignete Steuereinrichtung manuell zu dem Punkt gesteuert werden. Ebenso kann eine Hand-Auge-Kalibrierung durchgeführt werden.

Insbesondere kann das Verfahren weiterhin zumindest die folgenden Schritte umfassen:
- Bestimmen eines Güteparameters, der die Genauigkeit des Manipulators beim Anfahren der Soll-Pose angibt, wobei der Güteparameter insbesondere die Absolutgenauigkeit und/oder die Wiederholgenauigkeit des Manipulators angibt, und
- Widerholen der Schritte a) bis f), bis der Güteparameter einen vordefinierten Gütegrenzwert unterschritten hat.

Das Wiederholen der vorangehenden Schritte a) bis f) steigert die Genauigkeit bei der Kalibrierung, da auftretende Fehler gemittelt werden können. Insbesondere ist es möglich die Soll-Pose von unterschiedlichen Richtungen aus anzufahren, um eine höhere Genauigkeit zu erzielen. Dabei kann das Verfahren dann abgebrochen werden, wenn ein vordefinierter Güteparameter unterschritten wird, d. h. eine gewünschte Genauigkeit erreicht worden ist. Die Genauigkeit kann dabei beispielsweise die Wiederholgenauigkeit oder die Absolutgenauigkeit des Manipulators betreffen. Ebenso ist es möglich andere Genauigkeitsdefinitionen zur Bestimmung des Güteparameters heranzuziehen oder unterschiedliche Genauigkeitswerte zu kombinieren. Beispielsweise kann sich der Güteparameter anteilig aus einem ersten Faktor ergeben, der die Absolutgenauigkeit bestimmt und aus einem zweiten Faktor der die Wiederholgenauigkeit bestimmt.

Die medizinische Bildgebungsvorrichtung ist eine Röntgenbildgebungsvorrichtung, eine Ultraschallbildgebungsvorrichtung und/oder eine Magnetresonanzbildgebungsvorrichtung.

Diese medizinischen Bildgebungsvorrichtungen sind typischerweise in Operationssälen oder Behandlungszimmern vorhanden, sodass zum Kalibrieren des Manipulators keine weiteren herkömmlichen Messsysteme installiert oder in Betrieb genommen werden müssen. Zudem ermöglicht die Verwendung der medizinischen Bildgebungsvorrichtungen ein Kalibrieren des Manipulators mit hinreichender Genauigkeit, da das sich anschließende therapeutische oder diagnostische Verfahren mittels dieser medizinischen Bildgebungsvorrichtungen überwacht wird. Das therapeutische oder diagnostische Verfahren kann mit der Genauigkeit überwacht werden, welche die medizinische Bildgebungsvorrichtung bereitstellt. Dieselbe Genauigkeit kann dann beim Kalibrieren des Manipulators erzielt werden, so dass sichere Systeme erreicht werden.

Weiterhin kann die medizinische Bildgebungsvorrichtung dazu eingerichtet sein zweidimensionale und/oder dreidimensionale Bilder zu erstellen.

Werden von der medizinischen Bildgebungsvorrichtung zweidimensionale Bilder erstellt bzw. ist diese dazu eingerichtet, so kann gegebenenfalls das Erfassen von mehreren Bildern notwendigen sein, um die Ist-Pose des Manipulators eindeutig bestimmen zu können. Dreidimensionale Bilder haben dagegen den Vorteil, dass in der Regel ein dreidimensionales Bild reicht, um die Ist-Pose des Manipulators zu bestimmen.

Der Manipulator und/oder der Endeffektor können zumindest einen Marker umfassen, der dazu eingerichtet ist von der Bildgebungsvorrichtung erfasst zu werden, und wobei der Marker beim Erfassen des Bildes mit erfasst wird.

Marker, die am Manipulator und/oder Endeffektor angebracht sind oder mit diesen integral ausgebildet sind, haben den Vorteil, dass sie derart gestaltet sein können, um sicher von der medizinischen Bildgebungsvorrichtung erfasst zu werden. Ist die medizinische Bildgebungsvorrichtung beispielsweise eine röntgenbasierte medizinische Bildgebungsvorrichtung, wie beispielsweise ein C-Bogen oder ein Computertomograph (CT), so können die Marker Röntgenmarker sein, die im erfassten Bild eindeutig zu identifizieren sind. Insbesondere können die Marker so beschaffen sein, dass sie möglichst wenig Artefakte im erzeugten Bild hervorrufen, um eine genaue Erfassung der Marker zu ermöglichen und eine genaue Kalibrierung durchführen zu können. Im Falle magnetresonanzbasierten Bildgebungssystemen können die Marker beispielsweise flüssigkeitsgefüllte Objekte sein, wobei die Flüssigkeit beispielsweise Wasser und/oder ein Alkohol ist.

Der Marker kann überdies eine definierte geometrische Form aufweisen, welche die Bestimmung von Position und/oder Orientierung des Markers anhand des erfassten Bildes vereinfacht.

Weist der Marker geometrisch definierte Formen auf, so kann die Ist-Pose des Manipulators schnell und einfach bestimmt werden. Dabei ist der Marker vorzugsweise so ausgebildet, dass die Lage und Orientierung des Markers im Raum eindeutig bestimmt werden kann, wenn ein Bild, insbesondere genau ein Bild, erfasst wird.

Ein Beispiel für eine geeignete Form des Markers ist eine Dreiecksform, deren Seiten je eine unterschiedliche Länge aufweisen. Andere charakteristische Formen sind ebenso möglich. Insbesondere ist es nicht notwendig, dass der Marker eine physikalische Einheit bildet. Der Marker kann aus mehreren Untereinheiten zusammengesetzt sein, die in einer festen geometrischen Beziehung zueinander stehen. Im Falle eines Röntgenmarkers können beispielsweise drei einzelne Röntgenmarker auf einer entsprechenden Dreiecksstruktur angebracht sein oder dreieckförmig am Manipulator angebracht sein. Andere Anordnungen und geometrische Formen sind ebenso möglich. Beispielsweise kann es ausreichen bei rotationssymmetrischen Endeffektoren zwei Marker an der Longitudinalachse des Endeffektors anzuordnen, um dessen Position und Orientierung im Raum zu bestimmen. Ein rotationssymmetrischer Endeffektor ist beispielsweise ein Bohrer oder eine Biopsienadel.

Der Marker kann insbesondere integral mit dem Manipulator und/oder dem Endeffektor ausgebildet sein, und vorzugsweise integral mit einem Gehäuse des Manipulators und/oder des Endeffektors ausgebildet sein. Insbesondere kann der Manipulator und/oder der Endeffektor bzw. ein Teil und/oder eine bestimmte Struktur des Manipulators und/oder Endeffektors als Marker dienen.

Insbesondere das integrale Ausbilden des Markers mit dem Manipulator bzw. mit dem Endeffektor ermöglicht die Einsparung von Bauraum, sodass der Marker das diagnostische und/oder therapeutische Verfahren, das sich an das Kalibrierverfahren anschließt, nicht stört. Insbesondere können die Marker auch dazu eingerichtet sein, unterschiedliche Endeffektoren voneinander unterscheidbar zu machen. So kann beispielsweise ein erster Endeffektor, der ein Bohrer ist, einen ersten Marker umfassen und ein zweiter Endeffektor, der beispielsweise ein Schraubensetzwerkzeug ist, einen zweiten Marker umfassen, sodass beim Kalibrieren erkannt werden kann, welches Werkzeug/welcher Endeffektor der Manipulator augenblicklich führt.

Der Marker kann ebenso in einem Gehäuse des Manipulators und/oder des Endeffektors angeordnet sein, wobei das Gehäuse des Manipulators und/oder des Endeffektors für die Bildgebungseinrichtung transluzent und/oder transparent sein kann.

Insbesondere vorteilhaft ist es die Marker innerhalb des Gehäuses des Endeffektors/des Manipulators anzubringen, da so ein Stören durch den/die Marker verhindert bzw. vermieden wird. Dabei ist jedoch darauf zu achten, dass das Gehäuse das Erfassen der Marker nicht negativ beeinflusst. Daher sind transluzente oder transparente Gehäusematerialien vorteilhaft. Im Falle einer röntgenbasierten Bildgebungsvorrichtung können beispielsweise Kunststoffe verwendet werden, die für Röntgenstrahlen zumindest teilweise durchlässig sind.

Der Marker kann weiterhin lösbar mit dem Manipulator und/oder dem Endeffektor verbunden sein, wobei das Verfahren den folgenden Schritt umfassen kann: Anordnen und/oder Lösen zumindest eines Markers an dem Manipulator und/oder dem Endeffektor, wobei das Anbringen insbesondere mittels einer lösbaren Verbindung erfolgt.

Sind die Marker lösbar mit dem Manipulator verbunden, so ist es möglich diese nach dem Kalibrieren zu entfernen, um ein Stören der Marker bei der Ausführung des diagnostischen/therapeutischen Verfahren zu verhindern. In diesem Fall kann das Verfahren zum Kalibrieren die Verfahrensschritte umfassen: Anordnen zumindest eines Markers an dem Manipulator und/oder Endeffektor, so dass der Marker beim Bewegen des Manipulators vorzugsweise mitbewegt wird; und Lösen des Markers vom Manipulator und/oder Endeffektor nachdem die Kalibrierung durchgeführt wurde. Dabei ist das Lösen des Markers nicht zwingend notwendig, sondern kann als optionaler Verfahrensschritt ausgeführt werden.

Insbesondere können die Schritte a) bis f) für zumindest zwei unterschiedliche Soll-Positionen durchgeführt werden. Werden die Schritte a) bis f) des Verfahrens für unterschiedliche Soll-Positionen durchgeführt, so kann der Manipulator im gesamten Arbeitsbereich des Manipulators kalibriert werden und eine hohe Absolutgenauigkeit im gesamten Arbeitsbereich erzielt werden. Typischerweise werden als Soll-Posen unterschiedliche Soll-Posen verwendet, die über den gesamten Arbeitsbereich des Manipulators verteilt sind. Der Güteparameter, der gegebenenfalls zu unterschreiten ist, kann dabei auch für jede einzelne Soll-Pose unterschiedlich definiert sein. Insbesondere kann ein ortsabhängiger Güteparameter definiert sein. So kann beispielsweise der Manipulator in einem Zentrum, das typischerweise einer Operationsumgebung entspricht, eine sehr hohe Genauigkeit aufweisen, während im Randbereich des Arbeitsbereichs des Manipulators eine geringere Genauigkeit ausreichend ist.

Insbesondere kann der Manipulator beim Erfassen eines Bildes zumindest eines Teils des Manipulators und/oder zumindest eines Teils eines Endeffektors des Manipulators mittels der medizinischen Bildgebungsvorrichtung nicht stillstehen, sondern bewegt werden. In diesem Fall erfolgt das Erfassen des Bildes in dem Zeitpunkt, indem der Manipulator die Soll-Pose erreicht..

Wird das Kalibrieren nicht am stillstehenden Manipulator, sondern beim bewegten Manipulator durchgeführt, wie vorgehend beschrieben, so ist es möglich auch die Genauigkeit beim dynamischen Abfahren von Pfaden zu verbessern. Dabei wird die Abweichung von mehreren hintereinander folgenden Soll-Posen von den Ist-Posen bestimmt. Die erfassten Bilder können dabei aneinander gereihte Bilder sein, die ähnlich einem Video verarbeitet werden. Auch hier kommt es nicht darauf an, dass die erfassten Bilder tatsächlich als visuelle Eindrücke widergegeben werden, sondern es können vielmehr nur die Rohdaten der Bilder weiterverarbeitet werden.

Der zumindest eine Kalibrierungsparameter kann weiterhin von der Geschwindigkeit und/oder Beschleunigung des Manipulators beim Anfahren der Soll-Position abhängig sein. Ist der Kalibrierungsparameter neben dem reinen Offset d. h. der geometrischen Abweichung auch von Einflussgrößen wie Geschwindigkeit und Beschleunigung des Manipulators abhängig, so kann die erzielbare Genauigkeit weiter gesteigert werden, da auch Trägheitseffekte bei der Kalibrierung berücksichtigt werden. Die Berücksichtigung weiterer Einflussfaktoren zur Bestimmung des/der Kalibrierungsparameter ist ebenso möglich. Beispielsweise kann die Temperatur des Manipulators oder eines Teils des Manipulators berücksichtigt werden. Sind typische Verschleißkennwerte des Manipulators bekannt, so kann der Kalibrierungsparameter auch zeitabhängig sein und beispielsweise an eine Betriebsdauer des Manipulators angepasst sein.

Der Gütewertparameter kann im Betrieb des Manipulators fortlaufend aktualisiert werden und es kann eine Warnung ausgegeben werden, wenn der Gütewertparameter den vordefinierten Gütegrenzwert überschreitet. Wird der Güteparameter im Betrieb des Manipulators fortlaufend aktualisiert, so kann sichergestellt werden, dass eine gewünschte Genauigkeit ausreichend genau eingehalten wird. Wird der Güteparameter überschritten, so kann eine Warnung ausgegeben werden, die einen Benutzer dazu auffordert eine erneute Kalibrierung durchzuführen. Weiterhin kann das Manipulatorsystem nach dem Erkennen der Überschreitung des Güteparameters stillgesetzt werden oder beispielsweise mit verringerter Geschwindigkeit in einem Sicherheitsmodus gesteuert werden.

Weiterhin kann der/die Kalibrierungsparameter eine Eingangsgröße für ein Computermodell des Manipulators sein. Basierend auf dem Computermodell kann dann die an den Manipulator beauftragte Pose korrigiert werden, um beispielsweise eine höhere Absolutgenauigkeit zu erreichen. In einem einfachen, veranschaulichenden Beispiel berücksichtigt das Computermodell beispielsweise Offset-Werte der einzelnen Achsen des Manipulators als Kalibrierungsparameter. In einem komplexeren Computermodell können beispielsweise für jede Achse des Manipulators statische Abweichungen in sechs Freiheitsgraden bestimmt werden. Ebenso können dynamische Abweichungen, die beispielsweise von der Geschwindigkeit und/oder der Beschleunigung der jeweiligen

Achse(n) des Manipulators sind berücksichtigt werden. Auch der Einfluss von externen Kräften oder der Temperatur kann in das Computermodell mit einbezogen werden. Dazu kann das Manipulatorsystem zusätzliche Sensoren, vorzugsweise Kraft- und/oder Momenten-Sensoren und/oder Temperatursensoren umfassen. Weiterhin kann eine Last, die der Manipulator trägt, im Computermodell berücksichtigt werden. Dies kann unter Berücksichtigung der Parameter, Gewicht der Last, Massenschwerpunkt der Last und oder der gleichen erfolgen.

Der Manipulator kann ein mobiler Manipulator sein, der eine mobile Plattform umfasst, wobei an der mobilen Plattform vorzugsweise ein Marker angeordnet ist, der dazu eingerichtet ist von der Bildgebungsvorrichtung erfasst zu werden, und wobei der Marker beim Erfassen des Bildes mit erfasst wird.

Mobile Manipulatoren haben den Vorteil, dass sie ortsflexibel eingesetzt werden können. D.h. der eigentliche Manipulator ist auf/an einer mobilen Plattform angeordnet, welche sich (frei) im Raum bewegen kann. Dies ist insbesondere in engen Räumen vorteilhaft, in denen der Manipulator zusammen mit Menschen agiert, da der Manipulator frei positioniert werden kann und dem Menschen und/oder dem Manipulator so der bestmögliche Zugang zum Arbeitsbereich gewährt werden kann.

Der mobile Manipulator kann zumindest ein Kopplungsmittel umfassen, wobei der mobile Manipulator mittels des Kopplungsmittels ortsfest fixierbar ist. Um den mobilen Manipulator relativ zur Bildgebungsvorrichtung exakt positionieren und orientieren zu können, um eine genaue Kalibrierung durchführen zu können, kann der Manipulator Kopplungsmittel umfassen, mit denen er ortsfest fixierbar ist. Beispielsweise kann das Kopplungsmittel ein mechanisches Kopplungsmittel sein, mit dem der Manipulator an einem ortsfesten Gegenstand befestigt werden kann. Dabei kann das Kopplungsmittel beispielsweise als Vorsprung und ein komplementäres, ortsfestes Kopplungsmittel als korrespondierender Rücksprung ausgebildet sein, z.B. konus- oder kegelförmig, wobei der Konus formschlüssig in einen Gegenkonus passt. Andere geometrische Formen sind ebenso möglich. Insbesondere können die Kopplungsmittel miteinander verriegelt werden. Die Verriegelung kann mittels Formschluss und/oder Kraftschluss erfolgen. Beispielsweise können die Kopplungsmittel mit Magneten ausgestattet sein, sodass diese ineinander schnappen, wenn die Kopplungsmittel gekoppelt werden. Andere Kopplungsmittel, umfassend Verriegelungshebel, Schnapper und dergleichen sind ebenso denkbar.

Die Bildgebungsvorrichtung kann ein komplementäres Kopplungsmittel umfassen, welches mit dem Kopplungsmittel des mobilen Manipulators koppelbar ist, um den mobilen Manipulator relativ zur Bildgebungsvorrichtung zu fixieren. Ist die Bildgebungsvorrichtung mit einem komplementären Kopplungsmittel ausgestattet, so kann eine direkte Kopplung von mobilem Manipulator und Bildgebungsvorrichtung erfolgen, wodurch die Genauigkeit beim Kalibrieren weiter erhöht werden kann, da Bildgebungsvorrichtung und Manipulator exakt zueinander ausgerichtet sind. Insbesondere ist zu beachten, dass die Kopplungsmittel lösbar miteinander verbunden werden können und vorzugsweise Schnellkopplungsmittel sind, d.h. sie können ohne Werkzeugeinsatz miteinander gekoppelt und/oder entkoppelt werden.

Das Manipulatorsystem kann weiterhin zumindest einen ortsfesten Marker umfassen, der dazu eingerichtet ist von der Bildgebungsvorrichtung erfasst zu werden, wobei der Marker beim Erfassen des Bildes miterfasst wird. Ortsfeste Marker ermöglichen, wie die vorangehend beschriebenen Marker, welche am Manipulator angeordnet sind, einen räumlichen Bezug (Position und/oder Orientierung) zwischen Endeffektor und/oder Manipulator und dem ortsfesten Koordinatensystem herzustellen. Somit kann eine Kalibrierung durchgeführt werden. Insbesondere können die Marker auch an der Manipulatorbasis des Manipulators und/oder des mobilen Manipulators angeordnet sein.

Die Aufgabe wird weiterhin durch ein computerlesbares Medium gelöst, welches Programmbefehle umfasst, die die vorangehend beschriebene Steuereinrichtung dazu veranlassen, das vorangehend beschriebenen Verfahren auszuführen.

### Ausführliche Beschreibung der Figuren

Im Folgenden wird die Erfindung unter Bezugnahme auf die angefügten Figuren näher erläutert. Dabei zeigt
- Fig. 1: eine schematische Darstellung eines Manipulatorsystems;
- Fig. 2: eine schematische Darstellung eines weiteren Manipulatorsystems;
- Fig. 3: eine weitere Darstellung eines noch weiteren Manipulatorsystems, und
- Fig. 4: eine schematische Darstellung eines Verfahrens zum Kalibrieren.

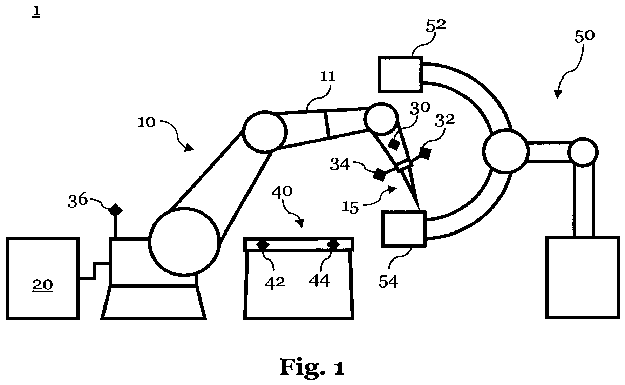

Insbesondere zeigt Fig. 1 ein Manipulatorsystem 1, welches einen Manipulator 10 umfasst, der für die Ausführung diagnostischer und/oder therapeutischer Verfahren eingerichtet ist. Der Manipulator 10 umfasst ein Gehäuse 11 und führt einen Endeffektor 15, der in Fig. 1 schematisch als Biopsienadel dargestellt ist. Andere Endeffektoren können ebenso mittels des Manipulators 10 geführt werden.

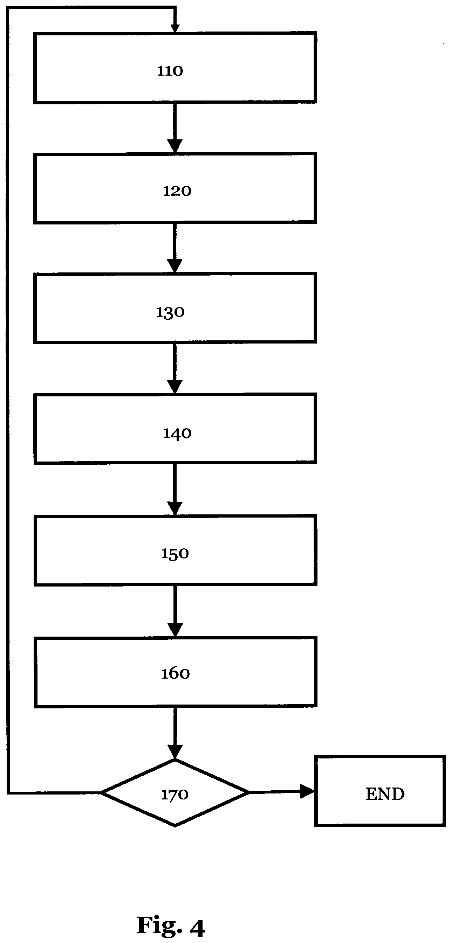

Der Manipulator wird mittels der Steuereinrichtung 20 gesteuert, welche sowohl Software als auch Hardwarekomponenten umfassen kann. Insbesondere ist die Steuereinrichtung dazu eingerichtet, den Manipulator entsprechend dem nachfolgend beschriebenen Verfahren zu steuern (vgl. Fig. 4). Der Manipulator umfasst weiterhin Marker 32, 30, 34, welche an seinem letzten Glied und/oder am Endeffektor angebracht sind. Insbesondere können die Marker 32, 30, 34 lösbar angeordnet sein.

Die Marker 32, 30, 34 sind so ausgestaltet, dass sie von der medizinischen Bildgebungsvorrichtung 50 erfasst werden können. Die medizinische Bildgebungsvorrichtung 50 kann jedwede medizinische Bildgebungsvorrichtung sein, welche in medizinischem Umfeld zum Einsatz kommt. Im gezeigten Beispiel ist sie als C-Bogen ausgeführt. Der C-Bogen besitzt eine Röntgenquelle 52 sowie eine Röntgenaufnahmeeinheit 54. Die Röntgenaufnahmeeinheit 54 erfasst die von der Röntgenquelle 52 emittierte Röntgenstrahlung um ein Bild zu erfassen.

Weiterhin kann das Manipulatorsystem eine Patientenliege 40 umfassen, welche mit ortsfesten Markern 42, 44 ausgestattet sein kann. Dem Manipulator 10 kann ein Marker 36 an dessen Manipulatorbasis zugeordnet sein. Das in Fig. 1 zu sehende Manipulatorsystem 1 umfasst vorzugsweise einen ortsfest angeordneten Manipulator 10 und eine ortsfest angeordnete Bildgebungsvorrichtungen 50, d.h. diese sind räumlich zueinander festgelegt.

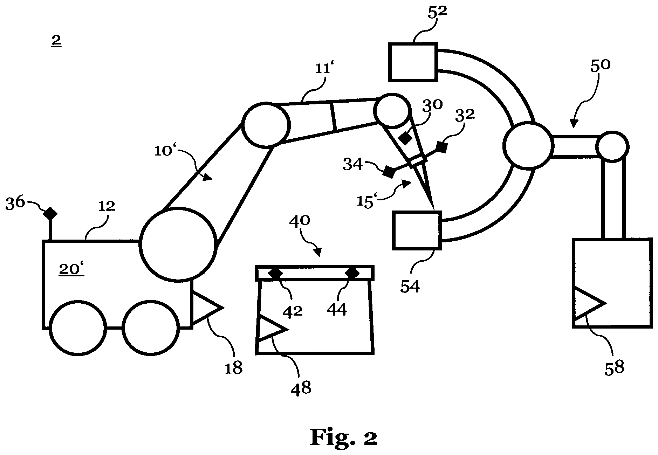

Fig. 2 zeigt ein weiteres Manipulatorsystem 2, das für diagnostische und/oder therapeutische Verfahren verwendet werden kann. Anstelle des Manipulators 10 der Fig. 1 wird hier ein mobiler Manipulator 10' verwendet. Der mobile Manipulator 10' umfasst ein Gehäuse 11', einen Endeffektor 15' und eine mobile Plattform 12 mittels derer sich der mobile Manipulator 10' frei im Raum bewegen kann. Dem Manipulator 10' ist eine Steuereinrichtung 20' zugeordnet, die dazu eingerichtet ist, den Manipulator zu steuern und das beschriebene Verfahren (vgl. Fig. 4) auszuführen.

Ebenso umfasst der Manipulator die vorangehend beschriebenen Marker 30, 32, 34, 36. Das Manipulatorsystem umfasst weiterhin eine Bildgebungsvorrichtung 50 (C-Bogen), welche über eine Röntgenquelle 52 und eine Röntgenaufnahmeeinheit 54 verfügt. Weiterhin kann dem Manipulatorsystem 2 eine Patientenliege 40 mit den Markern 42, 44 zugeordnet sein. Der mobile Manipulator 10' umfasst ein Kopplungsmittel 18, welches beispielsweise formschlüssig mit den komplementären Kopplungsmitteln 58 bzw. 48 der Bildgebungsvorrichtung 50 und/oder der Patientenliege 40 koppeln kann. Dadurch kann der Manipulator relativ zur Patientenliege 40 und/oder zur Bildgebungsvorrichtung 50 festgelegt werden, wodurch die Kalibrierung des Manipulators 10 vereinfacht wird.

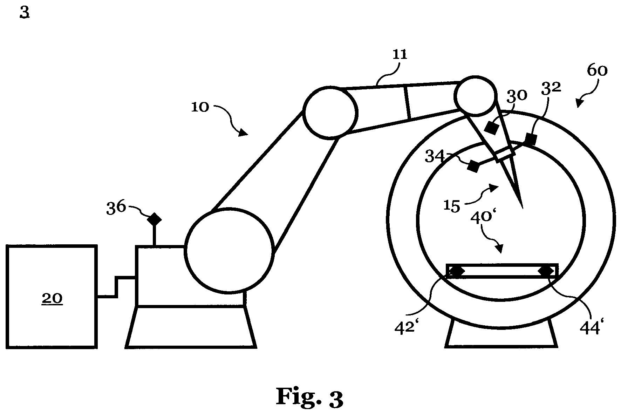

Fig. 3 zeigt ein weiteres Manipulatorsystem 3, welches für diagnostische und/oder therapeutische Verfahren einsetzbar ist. Neben dem bereits beschriebenen Manipulator 10 (vgl. Beschreibung zu Fig. 1) umfasst das Manipulatorsystem 3 eine Bildgebungsvorrichtung 60, welche beispielsweise ein Computertomograph oder ein Magnetresonanztomograph sein kann. In der Bildgebungsvorrichtung 60 ist eine Patientenliege 40' angeordnet, die entsprechende Marker 42', 44' umfasst. Die unter den Figuren 1-3 verwendeten gleichen Bezugszeichen beziehen sich jeweils auf die gleichen Komponenten der Manipulatorsysteme. Insbesondere können die Komponenten in den einzelnen Manipulatorsystemen 1, 2 und 3 ausgetauscht werden. So kann beispielsweise ein mobiler Manipulator 10' auch zusammen mit der Bildgebungseinheit 60 verwendet werden.

Fig. 4 zeigt ein Verfahren 100 zum Kalibrieren eines Manipulators 10, 10' eines diagnostischen und/oder therapeutischen Manipulatorsystems 1, 2, 3, wobei das Manipulatorsystem eine medizinische Bildgebungsvorrichtung 50, 60 umfasst. In einem ersten Verfahrensschritt 110 wird mittels des Manipulators zumindest eine Soll-Pose angefahren. Die Pose bezeichnet dabei Lage und Orientierung des Manipulators im Raum. Wird die Soll-Pose von unterschiedlichen Positionen angefahren, so muss das Verfahren mehrfach ausgeführt werden.

In einem zweiten Verfahrensschritt 120 wird zumindest ein Bild eines Teils des Manipulators und/oder zumindest eines Teils des Endeffektors des Manipulators mittels der medizinischen Bildgebungsvorrichtung erfasst, wenn der Manipulator die Soll-Pose angefahren hat oder diese gerade durchfährt. Dabei muss nicht ein Bild im Sinne eines Fotos oder eines visuell darstellbaren Bildes erzeugt werden, vielmehr reicht es aus, dass die Bildgebungsvorrichtung Rohdaten erfasst, die dazu geeignet sind, zu einem typischen medizinisch-diagnostischen Bild weiterverarbeitet zu werden.

Im dritten Verfahrensschritt 130 wird die Ist-Pose des Manipulators mittels des erfassten Bildes bzw. der erfassten Rohdaten bestimmt.

Im Verfahrensschritt 140 wird schließlich die Abweichung zwischen Soll-Pose und Ist-Pose des Manipulators bestimmt und in Schritt 150 ein Kalibrierungsparameter basierend auf dieser bestimmten Abweichung ermittelt. Der Kalibrierungsparameter kann auf rein geometrischen Parametern basieren und/oder weiterer Parameter wie der Anfahrgeschwindigkeit oder Beschleunigung des Manipulators, der Erwärmung des Manipulators, der Laufzeit des Manipulators und dergleichen.

In Verfahrensschritt 160 wird schließlich der Manipulator kalibriert. In Verfahrensschritt 170 kann überprüft werden, ob der Manipulator bereits eine gewünschte Genauigkeit erreicht hat, d.h. ob ein Güteparameter einen vordefinierten Gütegrenzwert unterschritten hat. Ist dies der Fall, kann das Verfahren beendet werden. Ist dies noch nicht der Fall, kann das Verfahren erneut ausgeführt werden. Insbesondere kann das Verfahren für mehrere Soll-Posen ausgeführt werden.

### Bezugszeichenliste

- 1, 2, 3: Manipulatorsystem
- 10: Manipulator
- 11: Gehäuse
- 12: mobile Plattform
- 15: Endeffektor
- 10': mobiler Manipulator
- 11': Gehäuse des mobilen Manipulators
- 15': Endeffektor des mobilen Manipulators
- 20: Steuereinrichtung
- 30, 32, 34, 36: Marker des Manipulators
- 18: Kopplungsmittel
- 40: Patientenliege
- 40': Patientenliege
- 42, 42', 44, 44': Marker der Patientenliege
- 50: Bildgebungsvorrichtung (C-Bogen)
- 52: Röntgenquelle
- 54: Röntgenerfassungseinheit
- 58: komplementäres Kopplungsmittel
- 48: komplementäres Kopplungsmittel
- 60: Bildgebungsvorrichtung (MRT)
- 100: Verfahren
- 110, 120, 130, 140, 150, 160, 170: Verfahrensschritte

## Patentansprüche

1. Verfahren (100) zum Kalibrieren eines Manipulators (10) eines diagnostischen und/oder therapeutischen Manipulatorsystems (1), wobei das Manipulatorsystem (1) zumindest eine medizinische Bildgebungsvorrichtung (50; 60) umfasst, und wobei das Verfahren zumindest die folgenden Schritte aufweist:
a) Anfahren (110) zumindest einer Soll-Pose mittels des Manipulators (10);
b) Erfassen (120) zumindest eines Bildes zumindest eines Teils des Manipulators (10) und/oder zumindest eines Teils eines Endeffektors (15) des Manipulators (10) mittels der medizinischen Bildgebungsvorrichtung (50; 60), wenn der Manipulator (10) die Soll-Pose angefahren hat;
c) Bestimmen (130) der Ist-Pose des Manipulators (10) mittels des erfassten Bildes;
d) Bestimmen (140) der Abweichung zwischen Soll-Pose und Ist-Pose des Manipulators (10);
e) Berechnen (150) zumindest eines Kalibrierungsparameters basierend auf der bestimmten Abweichung, und
f) Kalibrieren (160) des Manipulators,
**dadurch gekennzeichnet, dass** die medizinische Bildgebungsvorrichtung (50; 60) eine Röntgenbildgebungsvorrichtung (50), eine Ultraschallbildgebungsvorrichtung, eine Positronen-Emissions-Tomographie-Bildgebungsvorrichtung und/oder eine Magnetresonanzbildgebungsvorrichtung (60) ist.

2. Verfahren (100) nach Anspruch 1, wobei das Verfahren weiterhin zumindest die folgenden Schritte umfasst:
• Bestimmen eines Güteparameters, der die Genauigkeit des Manipulators (10) beim Anfahren der Soll-Pose angibt, wobei der Güteparameter insbesondere die Absolutgenauigkeit und/oder die Wiederholgenauigkeit des Manipulators angibt, und
• Wiederholen der Schritte a) bis f), bis der Güteparameter einen vordefinierten Gütegrenzwert unterschritten hat.

3. Verfahren (100) nach Anspruch 2, wobei der Gütewertparameter im Betrieb des Manipulators fortlaufend aktualisiert wird und eine Warnung ausgegeben wird, wenn der Gütewertparameter den vordefinierten Gütegrenzwert überschreitet

4. Verfahren (100) nach einem der vorhergehenden Ansprüche, wobei der Manipulator (10) und/oder der Endeffektor (15) zumindest einen Marker (30, 32, 34) umfasst, der dazu eingerichtet ist von der Bildgebungsvorrichtung (50; 60) erfasst zu werden, und wobei der Marker (30, 32, 34) beim Erfassen (120) des Bildes mit erfasst wird, wobei der Marker (30, 32, 34) bevorzugt eine definierte geometrische Form aufweist, welche die Bestimmung von Position und/oder Orientierung des Markers anhand des erfassten Bildes ermöglicht.

5. Verfahren (100) nach Anspruch 4, wobei der Marker (30, 32, 34) integral mit dem Manipulator (10) und/oder dem Endeffektor (15) ausgebildet ist, und insbesondere integral mit einem Gehäuse des Manipulators (10) und/oder des Endeffektors (15) ausgebildet ist.

6. Verfahren (100) nach einem der Ansprüche 4 bis 5, wobei der Marker (30, 32, 34) in einem Gehäuse des Manipulators (10) und/oder des Endeffektors (15) angeordnet ist, und wobei das Gehäuse des Manipulators (10) und/oder des Endeffektors (15) für die Bildgebungseinrichtung transluzent und/oder transparent ist.

7. Verfahren (100) nach einem der vorhergehenden Ansprüche, wobei die Schritte a) bis f) für zumindest zwei unterschiedliche Soll-Positionen durchgeführt werden.

8. Verfahren (100) nach einem der vorhergehenden Ansprüche, wobei der Manipulator beim Erfassen (120) eines Bildes zumindest eines Teils des Manipulators (10) und/oder zumindest eines Teils eines Endeffektors (15) des Manipulators (10) mittels der medizinischen Bildgebungsvorrichtung (50; 60) nicht stillsteht, und das Erfassen des Bildes erfolgt, wenn der Manipulator (10) die Soll-Pose erreicht hat.

9. Verfahren (100) nach einem der vorhergehenden Ansprüche, wobei der zumindest eine Kalibrierungsparameter von der Geschwindigkeit und/oder Beschleunigung des Manipulators beim Anfahren der Soll-Position abhängig ist.

10. Verfahren (100) nach einem der vorhergehenden Ansprüche, wobei der Manipulator (10) ein mobiler Manipulator ist, der eine mobile Plattform (12) umfasst, und wobei an der mobilen Plattform (12) vorzugsweise ein Marker (42) angeordnet ist, der dazu eingerichtet ist von der Bildgebungsvorrichtung (50; 60) erfasst zu werden, und wobei der Marker (30, 32, 34) beim Erfassen (120) des Bildes miterfasst wird.

11. Verfahren (100) nach Anspruch 10, wobei der mobile Manipulator (10) zumindest ein Kopplungsmittel umfasst, und wobei der mobile Manipulator mittels des Kopplungsmittels ortsfest fixierbar ist, wobei die Bildgebungsvorrichtung bevorzugt ein komplementäres Kopplungsmittel umfasst, welches mit dem Kopplungsmittel des mobilen Manipulators koppelbar ist, um den mobilen Manipulator relativ zur Bildgebungsvorrichtung zu fixieren.

12. Verfahren (100) nach einem der vorhergehenden Ansprüche, wobei das Manipulatorsystem (1) zumindest einen ortsfesten Marker (40) umfasst, der dazu eingerichtet ist von der Bildgebungsvorrichtung (50; 60) erfasst zu werden, und wobei der Marker (30, 32, 34) beim Erfassen (120) des Bildes miterfasst wird.

13. Computerlesbares Medium, welches Programmbefehle umfasst, die eine Steuereinrichtung, welche zumindest einen Prozessor und einen Datenspeicher umfasst, und wobei die Steuereinrichtung (20) dazu eingerichtet ist zumindest einen Manipulator (10) zu steuern dazu veranlassen, das Verfahren gemäß der Ansprüche 1 bis 12 auszuführen.

## Claims

1. Method (100) for calibrating a manipulator (10) of a diagnostic and/or therapeutic manipulator system (1), wherein the manipulator system (1) comprises at least one medical imaging device (50; 60), and wherein the method comprises at least the following steps:
a) starting (110) at least one desired pose by means of the manipulator (10);
b) acquiring (120) at least one image of at least part of the manipulator (10) and/or at least part of an end effector (15) of the manipulator (10) by means of the medical imaging device (50; 60) when the manipulator (10) has approached the desired pose;
c) determining (130) the actual pose of the manipulator (10) by means of the acquired image;
d) determining (140) the deviation between the target pose and the actual pose of the manipulator (10);
e) calculating (150) at least one calibration parameter based on the determined deviation, and
f) calibration (160) of the manipulator,
**characterized in that** the medical imaging apparatus (50; 60) is an X-ray imaging apparatus (50), an ultrasound imaging apparatus, a positron emission tomography imaging apparatus and/or a magnetic resonance imaging apparatus (60).

2. Method (100) according to claim 1, wherein the method further comprises at least the following steps:
- determining a quality parameter indicating the accuracy of the manipulator (10) when approaching the desired pose, the quality parameter indicating in particular the absolute accuracy and/or the repeatability of the manipulator, and
- repeat steps a) to f) until the quality parameter has fallen below a predefined quality limit.

3. Method (100) according to claim 2, wherein the quality value parameter is continuously updated during operation of the manipulator and a warning is issued if the quality value parameter exceeds the predefined quality limit.

4. Method (100) according to one of the preceding claims, wherein the manipulator (10) and/or the end effector (15) comprises at least one marker (30, 32, 34) adapted to be detected by the imaging device (50; 60), and wherein the marker (30, 32, 34) is also detected (120) when the image is captured, wherein the marker (30, 32, 34) preferably has a defined geometrical shape which allows the determination of position and/or orientation of the marker on the basis of the captured image.

5. Method (100) according to claim 4, wherein the marker (30, 32, 34) is formed integrally with the manipulator (10) and/or the end effector (15), and in particular is formed integrally with a housing of the manipulator (10) and/or the end effector (15).

6. Method (100) according to one of claims 4 to 5, wherein the marker (30, 32, 34) is arranged in a housing of the manipulator (10) and/or the end effector (15), and wherein the housing of the manipulator (10) and/or the end effector (15) is translucent and/or transparent for the imaging device.

7. Method (100) according to one of the preceding claims, wherein steps a) to f) are performed for at least two different desired positions.

8. Method (100) according to any of the foregoing claims, wherein the manipulator does not stand still during acquisition (120) of an image of at least part of the manipulator (10) and/or at least part of an end effector (15) of the manipulator (10) by means of the medical imaging device (50; 60), and acquisition of the image is performed when the manipulator (10) has reached the desired pose.

9. Method (100) according to one of the preceding claims, wherein the at least one calibration parameter is dependent on the speed and/or acceleration of the manipulator when moving to the desired position.

10. Method (100) according to one of the preceding claims, wherein the manipulator (10) is a mobile manipulator comprising a mobile platform (12), and wherein preferably a marker (42) is arranged on the mobile platform (12), which is adapted to be detected by the imaging device (50; 60), and wherein the marker (30, 32, 34) is also detected (120) when the image is acquired.

11. Method (100) according to claim 10, wherein the mobile manipulator (10) comprises at least one coupling means, and wherein the mobile manipulator is fixable in a stationary position by means of the coupling means, wherein the imaging device preferably comprises a complementary coupling means which is couplable to the coupling means of the mobile manipulator in order to fix the mobile manipulator relative to the imaging device.

12. Method (100) according to one of the preceding claims, wherein the manipulator system (1) comprises at least one stationary marker (40) adapted to be detected by the imaging device (50; 60), and wherein the marker (30, 32, 34) is co-detected (120) when the image is acquired.

13. A computer-readable medium comprising program instructions which cause a controller comprising at least one processor and a data memory, and wherein the controller (20) is adapted to control at least one manipulator (10) to execute the method according to claims 1 to 12.

## Revendications

1. Procédé (100) de calibrage d'un manipulateur (10) d'un système manipulateur diagnostique et/ou thérapeutique (1), dans lequel le système manipulateur (1) comprend au moins un dispositif d'imagerie médicale (50 ; 60), et dans lequel le procédé comprend au moins les étapes suivantes :
a) démarrage (110) d'au moins une pose souhaitée au moyen du manipulateur (10) ;
b) acquisition (120) d'au moins une image d'au moins une partie du manipulateur (10) et/ou d'au moins une partie d'un effecteur terminal (15) du manipulateur (10) au moyen du dispositif d'imagerie médicale (50 ; 60) lorsque le manipulateur (10) s'est approché de la pose souhaitée ;
c) déterminer (130) la pose réelle du manipulateur (10) au moyen de l'image acquise ;
d) déterminer (140) l'écart entre la pose cible et la pose réelle du manipulateur (10) ;
e) calculer (150) au moins un paramètre d'étalonnage basé sur l'écart déterminé, et
f) l'étalonnage (160) du manipulateur,
**caractérisé en ce que** l'appareil d'imagerie médicale (50 ; 60) est un appareil d'imagerie à rayons X (50), un appareil d'imagerie à ultrasons, un appareil d'imagerie par tomographie par émission de positrons et/ou un appareil d'imagerie par résonance magnétique (60).

2. Méthode (100) selon la revendication 1, dans laquelle la méthode comprend en outre au moins les étapes suivantes :
- déterminer un paramètre de qualité indiquant la précision du manipulateur (10) à l'approche de la pose souhaitée, le paramètre de qualité indiquant en particulier la précision absolue et/ou la répétabilité du manipulateur, et
- répéter les étapes a) à f) jusqu'à ce que le paramètre de qualité soit tombé en dessous d'une limite de qualité prédéfinie.

3. Méthode (100) selon la revendication 2, dans laquelle le paramètre de valeur de qualité est continuellement mis à jour pendant le fonctionnement du manipulateur et un avertissement est émis si le paramètre de valeur de qualité dépasse la limite de qualité prédéfinie.

4. Procédé (100) selon l'une des revendications précédentes, dans lequel le manipulateur (10) et/ou l'effecteur final (15) comprend au moins un marqueur (30, 32, 34) adapté pour être détecté par le dispositif d'imagerie (50 ; 60), et dans lequel le marqueur (30, 32, 34) est également détecté (120) lorsque l'image est capturée, dans lequel le marqueur (30, 32, 34) a de préférence une forme géométrique définie qui permet de déterminer la position et/ou l'orientation du marqueur sur la base de l'image capturée.

5. Procédé (100) selon la revendication 4, dans lequel le marqueur (30, 32, 34) est formé d'un seul tenant avec le manipulateur (10) et/ou l'effecteur final (15), et en particulier est formé d'un seul tenant avec un bo tier du manipulateur (10) et/ou de l'effecteur final (15).

6. Procédé (100) selon l'une des revendications 4 à 5, dans lequel le marqueur (30, 32, 34) est disposé dans un bo tier du manipulateur (10) et/ou de l'effecteur final (15), et dans lequel le bo tier du manipulateur (10) et/ou de l'effecteur final (15) est translucide et/ou transparent pour le dispositif d'imagerie.

7. Procédé (100) selon l'une des revendications précédentes, dans lequel les étapes a) à f) sont effectuées pour au moins deux positions souhaitées différentes.

8. Procédé (100) selon l'une des revendications précédentes, dans lequel le manipulateur ne reste pas immobile pendant l'acquisition (120) d'une image d'au moins une partie du manipulateur (10) et/ou d'au moins une partie d'un effecteur terminal (15) du manipulateur (10) au moyen du dispositif d'imagerie médicale (50 ; 60), et l'acquisition de l'image est effectuée lorsque le manipulateur (10) a atteint la position souhaitée.

9. Méthode (100) selon l'une des revendications précédentes, dans laquelle le au moins un paramètre d'étalonnage dépend de la vitesse et/ou de l'accélération du manipulateur lorsqu'il se déplace vers la position souhaitée.

10. Procédé (100) selon l'une des revendications précédentes, dans lequel le manipulateur (10) est un manipulateur mobile comprenant une plate-forme mobile (12), et dans lequel de préférence un marqueur (42) est disposé sur la plate-forme mobile (12), qui est adapté pour être détecté par le dispositif d'imagerie (50 ; 60), et dans lequel le marqueur (30, 32, 34) est également détecté (120) lorsque l'image est acquise.

11. Procédé (100) selon la revendication 10, dans lequel le manipulateur mobile (10) comprend au moins un moyen de couplage, et dans lequel le manipulateur mobile peut être fixé dans une position stationnaire au moyen du moyen de couplage, dans lequel le dispositif d'imagerie comprend de préférence un moyen de couplage complémentaire qui peut être couplé au moyen de couplage du manipulateur mobile afin de fixer le manipulateur mobile par rapport au dispositif d'imagerie.

12. Procédé (100) selon l'une des revendications précédentes, dans lequel le système de manipulateur (1) comprend au moins un marqueur fixe (40) adapté pour être détecté par le dispositif d'imagerie (50; 60), et dans lequel le marqueur (30, 32, 34) est co-détecté (120) lors de l'acquisition de l'image.

13. Support lisible par ordinateur comprenant des instructions de programme qui font qu'un contrôleur comprenant au moins un processeur et une mémoire de données, et dans lequel le contrôleur (20) est adapté pour commander au moins un manipulateur (10) pour exécuter la méthode selon les revendications 1 à 12.
